Europäisches Patentamt

European Patent Office

Office européen des brevets

(11) Veröffentlichungsnummer: **0 263 401 B1**

# EUROPÄISCHE PATENTSCHRIFT

(45) Veröffentlichungstag der Patentschrift: **12.06.91**

(51) Int. Cl.⁵: **G01N 33/577**, G01N 33/541, //G01N33/574,G01N33/76

(21) Anmeldenummer: **87114166.9**

(22) Anmeldetag: **29.09.87**

(54) **Immunometrisches Bestimmungsverfahren.**

(30) Priorität: **02.10.86 DE 3633497**

(43) Veröffentlichungstag der Anmeldung:
**13.04.88 Patentblatt 88/15**

(45) Bekanntmachung des Hinweises auf die
Patenterteilung:
**12.06.91 Patentblatt 91/24**

(84) Benannte Vertragsstaaten:
**AT BE CH DE ES FR GB GR IT LI LU NL SE**

(56) Entgegenhaltungen:
**EP-A- 0 062 892**
**WO-A-82/01773**
**WO-A-85/00663**
**DE-A- 3 130 834**

(73) Patentinhaber: **HOECHST AKTIENGESELL-SCHAFT**
**Postfach 80 03 20**
**W-6230 Frankfurt am Main 80(DE)**

(72) Erfinder: **Schnorr, Gerd, Dr.**
**Auf dem Lattigkopf 23**
**W-6368 Bad Vilbel(DE)**
Erfinder: **Strecker, Helmut, Dr.**
**verstorben**
**Weceased(DE)**
Erfinder: **Molz, Peter, Dr.**
**Kaiser Wilhelm-Ring 44**
**W-6500 Mainz(DE)**
Erfinder: **Simons, Guido, Dr.**
**Talstr. 24**
**W-6507 Ingelheim am Rhein(DE)**
Erfinder: **Skrzipczyk, Heinz Jürgen, Dr.**
**Am Schellberg 16**
**W-6232 Bad Soden am Taunus(DE)**

## Beschreibung

Gegenstand der Erfindung ist ein immunometrisches Verfahren zur Bestimmung einer mindestens zwei Antikörperbindungsstellen aufweisenden antigenen Substanz.

Es ist bekannt, daß zum qualitativen und quantitativen Nachweis von Antigenen in immer größerem Maß immunometrische Methoden herangezogen werden. Diese Methoden beruhen auf der Bilding eines Komplexes des Antigens mit einem oder mehreren Antikörpern, wobei einer der Bindungspartner markiert ist. Dadurch ist es möglich festzustellen, ob und in welcher Menge sich ein Komplex aus dem Antigen und einem oder mehreren Antikörpern gebildet hat. Entscheidende Verbesserungen der immunometrischen Bestimmungsverfahren gelangen mit der Einführung monoklonaler Antikörper (Milstein und Köhler, 1975), deren Anwendung in immunometrischen Assays in der deutschen Offenlegungsschrift 31 30 834 eingehend beschrieben ist.

Die immunometrischen Bestimmungsmethoden können in zwei Haupttypen unterteilt werden, je nachdem, ob das Antigen oder der Antikörper markiert ist. Der markierte Bindungspartner wird stets im Überschuß verwendet.

Besonderes Interesse haben immunometrische Verfahren gefunden, bei denen einer der verwendeten Antikörper markiert ist. Dabei wird das Antigen in Form eines ternären Komplexes sandwich-artig gebunden und nach der Inkubation der nicht gebundenen markierte Antikörper mit dem Reaktionsgemisch durch Dekantieren oder Auswaschen entfernt. Diese Ausführungsformen werden je nach der Art der Markierung als zweiseitiger immuno-radiometrischer Assay (IRMA), als immuno-enzymometrischer Assay (IEMA) oder immuno-chemiluminometrischer Assay (ICMA) bezeichnet. In der Regel ist dabei der unmarkierte Antikörper an eine feste Phase gebunden.

Die vorstehend genannten Sandwich-Assays können in verschiedenen Varianten zur Ausführung kommen, die sich durch die zur Bilding des ternären Komplexes führenden Reaktionsschritte unterscheiden. Man kann entweder in einem Eintopfverfahren das Antigen mit dem markierten und dem unmarkierten Antikörper gleichzeitig zusammengeben, man kann aber auch sequentiell vorgehen und daß Antigen zunächst mit dem unmarkierten und nach einer ausreichenden Inkubationszeit dann auch mit dem markierten Antikörper reagieren lassen. Schließlich können diese Reaktionsschritte auch in der umgekehrten Reihenfolge durchgeführt werden.

Die auf der Verwendung von markierten Antikörpern beruhenden Sandwich-Assays haben gegenüber den unter Verwendung markierter Antigene durchgeführten Assays entscheidende analytische Vorteile: Es ist möglich, die Antikörper im Überschuß einzusetzen und durch die Erhöhung ihrer Konzentration das Gleichgewicht in Richtung auf die Bildung des ternären Komplexes zu verschieben. Damit können auch noch geringe Antigenmengen gebunden und an die Markierung gekoppelt werden. Da außerdem das von der Markierung ausgesendete Signal der Antigenkonzentration direkt proportional ist, können auch die durch niedrige Antigenkonzentrationen verursachten schwachen Signale noch deutlich erkannt werden. Aus diesen Gründen sind die mit markierten Antikörpern arbeitenden Sandwich-Assays wesentlich empfindliche als Assays, die auf der Verwendung markierter Antigene beruhen.

Schließlich spricht für die mit markierten Antikörpern arbeitenden Sandwich-Assays auch der wesentlich größere dynamische Meßbereich, d.h. es gibt einen größeren Bereich, in welchem sich bei einer Änderung der Konzentration des Antigens auch die Stärke des ausgesendeten Signals des ternären Komplexes empfindlich ändert. Außerdem ist es für die routinemäßige Anwendung kommerzieller immunometrischer Bestimmungsverfahren noch von Bedeutung, daß sie möglichst kurze Inkubationszeiten gestatten. Das ist dann gewährleistet, wenn höhere Reagenzkonzentrationen eingesetzt werden können, wie es bei dem Einsatz von markierten Antikörpern in Sandwich-Assays der Fall ist.

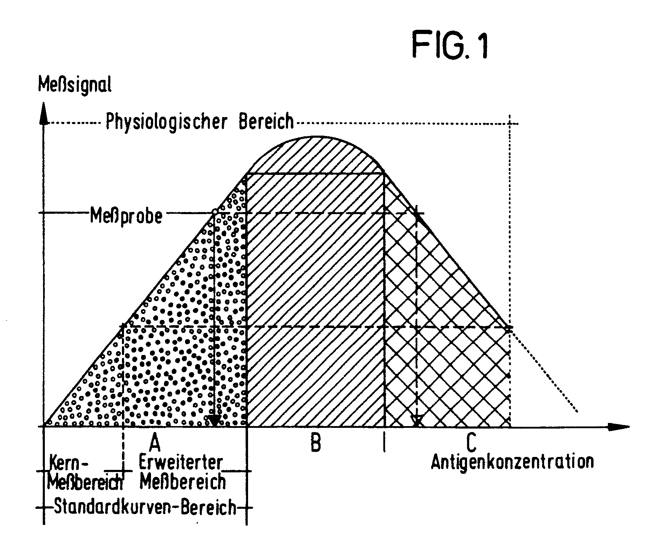

Die DE-A 31 30 834 beschreibt die obengeschilderten immunometrischen Verfahren, jedoch auf monoklonale Antikörper beschränkt. Ebenso wurde nicht das Problem gelöst, daß das gemessene Signal nicht im gesamten Konzentrationsbereich eindeutig einer bestimmten Antigenmenge zuzuordnen ist. Vielmehr wird bei besonders hohen Antigenkonzentrationen ein schwächeres Signal erhalten als bei niedrigeren Antigenkonzentrationen. Das wird in Figur 1 dargestellt, in der die Stärke des ausgesendeten Meßsignals in Abhängigkeit von der Antigenkonzentration angegeben ist. Diese Dosis-Wirkungs-Kurve ist in drei Bereiche eingestellt:

a) Der Standardkurvenbereich (A), der aus einem Kernmeßbereich und einem erweiterten Meßbereich besteht;

b) dem Bereich (B), in dem das Meßsignal größer als das Meßsignal des höchsten Standards;

c) der Bereich (C), in dem das Meßsignal dem Standardkurvenbereich entspricht, obwohl die Antigenkonzentration um ein Vielfaches höher ist.

Figur 1 zeigt, daß nur im Kernmeßbereich mit einer einzigen Messung eindeutige Ergebnisse erhalten werden können. Dagegen können die im erweiterten Meßbereich erhaltenen Ergebnisse zwei ganz unterschiedlichen Antigenkonzentrationen zugeordnet werden. Dieses Phänomen wird als "high-dose-hook"-Effekt bezeichnet und hat zur Folge, daß der Antigengehalt in der Probe, wenn er der bei einer viel niedrigeren Konzentration endenden Standardkurve zugeordnet wird, stark unterschätzt wird.

Dagegen sind die im Bereich (B) gemessenen Signale, die größer als der höchste Standard sind, eindeutig und können nach entsprechender Verdünnung exakt angegeben werden.

Der "high-dose-hook"-Effekt wird praktisch immer beobachtet, wenn das Bestimmungsverfahren als Eintopf-Verfahren durchgeführt wird, er tritt aber auch bei einer sequentiellen Inkubation auf.

Als Ursache werden einerseits veränderte Affinitätskonstanten diskutiert, die bei den hohen Antigenkonzentrationen aufgrund sterischer Effekte ein späteres Ablösen des markierten Antikörpers bewirken, andererseits gilt beim Eintopf-Verfahren nicht mehr die Reagenzüberschußsituation des markierten Reagenzes im Vergleich zum hoch konzentrierten Antigen. Dies führt dazu, daß das Antigen nicht nur alle Bindungsplätze des an die Festphase gebundenen markierten Antikörpers, sondern auch die Bindungsplätze des markierten Antikörpers besetzt und dadurch die Bildung eines ternären Komplexes unmöglich wird. Diese Konkurrenz ist bei niedrigeren Antigenkonzentrationen nicht vorhanden, so daß der ternäre Komplex entstehen und ein der Antigenkonzentration entsprechendes Meßsignal aussenden kann.

Auch bei den Bestimmungsmethoden mit sequentieller Inkubation kann der "high-dose-hook"-Effekt auftreten, wenn es nicht gelingt, die das Antigen enthaltende Flüssigkeitsprobe nach der Reaktion mit dem immobilisierten, unmarkierten Antikörper vor der Zugabe des markierten Antikörpers quantitativ zu entfernen. Es besteht dann die Gefahr, daß hohe Konzentrationen von ungebundenem Antigen im Teströhrchen zurückbleiben, die den anschließend zugegebenen markierten Antikörper blockieren. Die quantitative Entfernung von Resten der Flüssigkeitsprobe vor Zugabe des markierten Antikörpers durch Auswaschen der Festphase ist deshalb schwierig, weil bei jedem Waschschritt auch eine Dissoziation des aus Antigen und unmarkiertem Antikörper gebildeten Komplexes auftritt, die natürlich möglichst verhindert werden soll. Deshalb können nicht beliebig viele Waschschritte vorgenommen werden.

Der Nachweis eines durch den "high-dose-hook"-Effekt verfälschten Meßergebnisses wurde nach dem Stand der Technik bisher in folgender Weise geführt:

a) Die zu untersuchende Flüssigkeitsprobe wurde bei zwei verschiedenen Verdünnungen gemessen. Verstärkte sich dabei das Meßsignal trotz Verdünnung, konnte daraus auf das Vorliegen des "high-dose-hook"-Effektes geschlossen werden, was aus einer Betrachtung von Figur 1 sofort deutlich wird;

b) Es wurde eine Bestimmungsmethode mit sequentieller Inkubation gewählt und dabei durch zahlreiche Waschschritte sichergestellt, daß vor Zugabe des markierten Antikörpers kein freis Antigen mehr vorhanden war.

Beide Bestimmungsmethoden sind mit erheblichem Aufwand verbunden.

Durch das erfindungsgemäße Bestimmungsverfahren wird nun ein Weg zur Erkennung des "high-dose-hook"-Effektes gezeigt, bei dem man in einer ersten Messung die Antigenkonzentration mit Hilfe des Sandwich-Verfahrens in Flüssigkeitsproben ermittelt, die das Antigen in allen physiologisch möglichen Konzentrationen enthalten können und nur bestimmte ausgewählte Proben danach einem nachgeschalteten Schnelltest unterzieht. Damit werden für alle Proben eindeutige Ergebnisse bei geringem technischen Aufwand erhalten.

Gegenstand der Erfindung ist deshalb ein immunometrisches Bestimmungsverfahren für eine mindestens zwei Antikörperbindungsstellen aufweisende antigene Substanz in einer Probe, für die eine Antigenkonzentration in einem Bereich festgestellt wurde, der bis zu den Konzentrationen reicht, die noch mit genauer Präzision gemessen werden können (erweiterter Meßbereich), bei dem man eine die antigene Substanz (a) enthaltende Flüssigkeitsprobe mit einem auf einer Festphase immobilisierten, unmarkierten, für (a) spezifischen Antikörper (b) und einem weiteren, für (a) spezifischen, markierten Antikörper (c) sequentiell oder in einem Schritt inkubiert, wobei sich ein festphasengebundener, ternärer Komplex aus (a), (b) und (c) bildet, der ein nachweisbares Signal entsprechend der Menge von (a) aussendet, wobei man in einer ersten Messung die Antigenkonzentration über den gesamten Meßbereich, bestehend aus einem Bereich, in dem bei physiologischen Proben die dose-response Zuordnung eindeutig ist (Kernmeßbereich) und dem Bereich, der bis zu den Konzentrationen reicht, die noch mit genauer Präzision gemessen werden können (erweitertem Meßbereich) ermittelt, dadurch gekennzeichnet, daß man die Probe, für die eine Antigenkonzentration im erweiterten Meßbereich festgestellt wurde, nach Erhöhung der Menge des Antigens und/oder eines Antigenäquivalents einer zweiten Messung unterwirft.

Zur Erzeugung einer Signaldifferenz bei der zweiten Messung ergeben sich folgende Möglichkeiten:

a) Man kann vor Durchführung der zweiten Messung in die Probe zusätzliches Antigen oder ein Antigen-Äquivalent geben. Als Antigen-Äquivalent kann ein anti-idiotypischer Antikörper dienen;

b) Man kann die Messung mit einem größeren Probenvolumen durchführen.

EP 0 263 401 B1

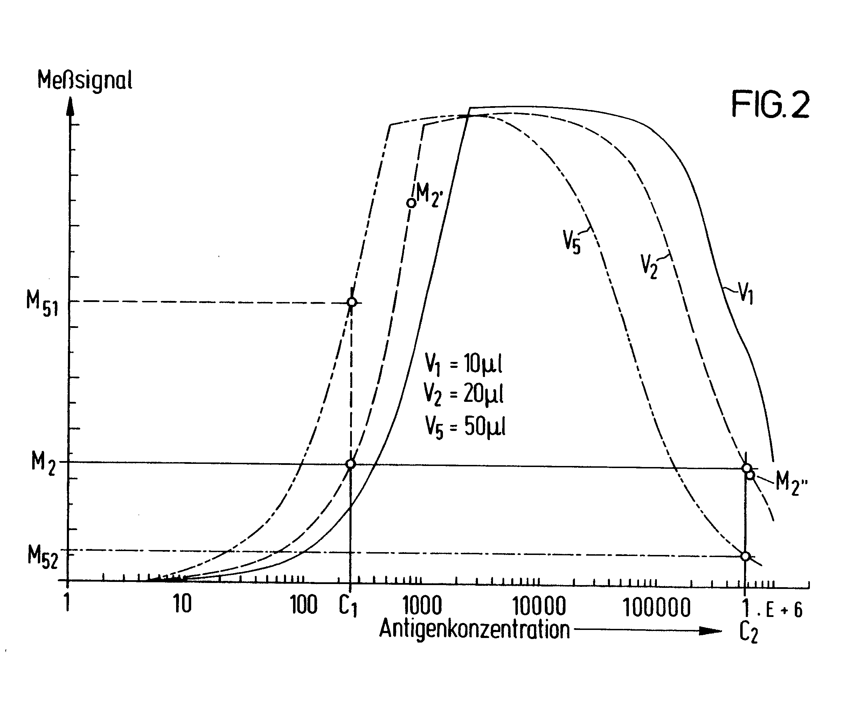

Die dabei auftretenden Effekte werden durch Figur 2 verdeutlicht. Dort ist das Meßsignal in Abhängigkeit von der Antigenkonzentration für unterschiedliche Probenvolumina dargestellt. Es zeigt sich, daß mit abnehmendem Probenvolumen die Kurve nach rechts verschoben wird. Führt man die Bestimmung mit dem mittleren Probenvolumen $V_2$ aus, so erhält man für die Antigenkonzentrationen $C_1$ und $C_2$ das Meßsignal $M_2$ d.h., die beiden Proben sind nicht unterscheidbar, obwohl ihre Antigenkonzentrationen weit auseinanderliegen. Wird nun die Antigenkonzentration erhöht, dann verschiebt sich das Signal von $C_1$, $M_2$ drastisch zu höheren Werten ($M_2'$), während das zu $C_2$ gehörende Signal $M_2''$ nur geringfügig abgeschwächt wird. Jetzt kann durch den Vergleich des Signals der ersten Messung mit dem Signal der zweiten Messung der Probe ein eindeutiger Konzentrationsbereich zugeordnet werden d.h., der "high-dose-hook"-Effekt kann entweder bestätigt oder ausgeschlossen werden.

Wenn das Antigen nicht leicht verfügbar ist, kann an seiner Stelle auch sein antigenes Äquivalent, eine anti-idiotypischer Antikörper, eingesetzt werden. Da ein anti-idiotypischer Antikörper im Unterschied zum Antigen nur eine Bindungsstelle für den Antikörper aufweist, kann er die Ausbildung des ternären Komplexes behindern. Deshalb kommt es in diesem Fall zu einer Abschwächung des Meßsignals bei niedrigen Antigenkonzentrationen, während sich bei hohen Antigenkonzentrationen kaum etwas ändert (vgl. hierzu auch Beispiel 3).

Der "high-dose-hook"-Effekt kann aber dadurch erkannt werden, daß man für die zweite Messung durch Erhöhung des Probenvolumens die Menge des nachzuweisenden Antigens steigert. Führt man die zweite Messung bei dem in Figur 2 gezeigten Probenvolumen $V_5$ durch, dann erhält man bei der Konzentration $C_1$ das Signal $M_{51}$ und bei der Konzentration $C_2$ das Signal $M_{52}$. Diese Signale unterscheiden sich in ihrer Stärke deutlich von den bei der ersten Messung erhaltenen Signalen. Damit ist ein qualitativer Schnelltest möglich, der eine eindeutige Aussage erlaubt, ob ein "high-dose-hook"-Effekt vorliegt.

Beiden vorstehend genannten Bestimmungsmethoden ist gemeinsam, daß eine Erhöhung der Konzentration von Antigen oder der Zusatz von anti-idiotypischem Antikörper vor der zweiten Messung zu einer Verschiebung des Meßsignals führt. Verstärkt sich das Meßsignal bei der Erhöhung der Antigenkonzentration oder schwächt es sich ab bei Zugabe von anti-idiotyptischen Antikörper, dann kann kein "high-dose-hook"-Effekt vorliegen. Verändert sich das Meßsignal dagegen trotz Erhöhung der Antigenkonzentration oder Zugabe des anti-idiotypischen Antikörpers nur unwesentlich, dann muß auf einen "high-dose-hook"-Effekt geschlossen werden.

In der Praxis wird die erfindungsgemäße Bestimmungsmethode nun in der Weise angewendet, daß man die bei der ersten Messung erhaltenen Meßergebnisse mit einer Standardkurve vergleicht. Die Standardkurve ist in Abhängigkeit von der Antigenkonzentration in drei Meßbereiche eingeteilt, den Kernmeßbereich, den erweiterten Meßbereich und den Außenbereich.

Der Kernmeßbereich ist als der Bereich definiert, in dem bei physiologischen Proben die dose-response Zuordnung eindeutig ist. Der erweiterte Meßbereich wird nach analytischen Kriterien festgelegt und reicht bis zu den Konzentrationen, die noch mit genauer Präzision gemessen werden können.

Liegen die erhaltenen Werte im Kernmeßbereich, dann sind sie eindeutig und können als endgültige Ergebnisse ausgewertet werden. Liegt das Meßergebnis dagegen im erweiterten Meßbereich, dann muß ein Test auf Vorliegen des "high-dose-hook"-Effektes durchgeführt werden. Verläuft dieser Test negativ, dann ist das in der ersten Messung erhaltene Ergebnis als eindeutig anzusehen. Liegt dagegen ein "high-dose-hook"-Effekt vor, dann muß diese Probe solange verdünnt werden, bis in einer weiteren Messung ein eindeutiges Ergebnis erhalten werden kann.

Werden in der ersten Messung hohe Meßsignale außerhalb des erweiterten Meßbereichs gefunden, dann kann kein "high-dose-hook"-Effekt vorliegen. Die Probe muß aber verdünnt werden, bis in einer zweiten Messung ein zuverlässiger Wert erhalten werden kann.

Das erfindungsgemäße Bestimmungsverfahren läßt sich besonders vorteilhaft dann durchführen, wenn der auf einer Festphase immobilisierte, unmarkierte Antikörper ein polyklonaler oder monoklonaler Antikörper ist. Er kann auf der Festphase adsorptiv oder kovalent gebunden sein. Als Festphase kann entweder ein makroskopischer Träger wie Kunststoffröhrchen, Mikrotiter-Platten oder verschiedenartig geformte Kunststoffkörper, z.B. Kugeln oder Propeller verwendet werden. Bei der Festphase kann es sich aber auch um eine Suspension von mikroskopischen Teilchen handeln, deren Größe im allgemeinen zwischen 0,1 $\mu$m und 10 $\mu$m liegt. Diese mikroskopischen Teilchen können aus Polystyrol, Polypropylen, Polymethylmethacrylat, Polyacrylamid, Glas, Siliziumdioxid oder Sepharose bestehen.

Auch der zweite, der markierte Antikörper, sollte ein monokloner oder polyklonaler Antikörper sein. Er kann durch ein radioaktives Isotop wie Jod-125 oder Kobalt-57 markiert sein, welches nach Ausbildung des ternären Komplexes durch Messung der radioaktiven Strahlung den gehalt an Antigen durch Vergleich mit einer unter identischen Bedingungen erstellten Standardkurve erlaubt. Die Markierung kann aber auch durch ein Enzym wie Peroxidase, erfolgen, welches nach Ausbildung des ternären Komplexes durch Messung der

4

EP 0 263 401 B1

Enzymaktivität den Gehalt an Antigen erkennen läßt. Schließlich können auch fluoreszierende Gruppen wie Fluorescein, oder chemilumineszierende Gruppen wie Acridinium-Derivate, als Indikatoren an den Antikörper gebunden sein.

Mit dem erfindungsgemäßen immunometrischen Bestimmungsverfahren können physiologische Konzentrationen von antigenen Substanzen über einen Bereich von mindestens 5 Zehnerpotenzen sicher ermittelt werden. Die Methode ist einsetzbar zur Bestimmung der unterschiedlichsten antigenen Substanzen, z.B. für das humane thyreoideastimulierende Hormon (hTSH) oder für die onkofetalen Proteine wie Alfa-Fetoprotein, h-Choriongonadotropin oder das carcinoembrionale Antigen (CEA).

Das neue erfindungsgemäße immunometrische Bestimmungsverfahren ist zuverlässig, technisch außerordentlich einfach, schnell und robust. Es kann bei einer Vielzahl von kommerziellen Sandwich-Bestecken angewendet werden, weil keine Veränderung der Komponenten erforderlich ist.

Die Erfindung wird durch die nachfolgenden Beispiele näher erläutert:

Beispiel 1

Radioimmunologische Bestimmung von Alfa-Fetoprotein in Patientenserum mit Hilfe eines auf monoklonalen Antikörpern basierenden zweiseitigen IRMA und der Technik der zweistufigen Verfahrensdurchführung.

Das onkofetale Alfa-Fetoprotein (AFP) besitzt Bedeutung in zwei Indikationsgebieten:

1. Als Parameter für das Screening auf Neuralrohrdefekte in Feten (Bestimmung in mütterlichem Serum innerhalb der 15. - 22. Schwangerschaftswoche. Zur Bestätigiung wird AFP zusätzlich im Fruchtwasser bestimmt).

2. Als Tumormarker - vor allem in der Verlaufskontrolle von trophoblastischen Tumoren.

Die Normal- bzw. pathologischen Bereiche sind bei diesem Parameter für die beiden Indikationen sehr unterschiedlich. Im unter 1. bezeichneten Indikationsgebiet überstreichen die Serumproben einen Bereich von 5 - 8000 IU AFP/ml; die Fruchtwasserproben den Bereich von 1000 - 250.000 IU AFP/ml. Für die letztgenannten Proben kann eine 1/100 Vorverdünnung obligatorisch vorgeschrieben werden, so daß dann Werte von 10 - 2.500 IU AFP/ml zu erwarten sind. Der diagnostische Entscheidungsbereich ("cut off") liegt bei ca. 100 IU AFP/ml.

In der Onkologie werden bei tumormarkernegativen Proben Werte unter 10 IU AFP/ml gefunden, während hochpathologische Proben mehr als 1000 000 IU AFP/ml aufweisen können. Somit ist das beschriebene Problem der Uneindeutigkeit infolge des "high-dose-hook"-Effektes nur für die letztere Indikation von Bedeutung. Jedoch sollen in der klinischen Routinediagnostik aus Rationalisierungsgründen beide Indikationen mit einem Assay abgedeckt werden. Hierbei ist sogar mitunter unbekannt, welchem Indikationsgebiet die Patientenprobe zuzuordnen ist.

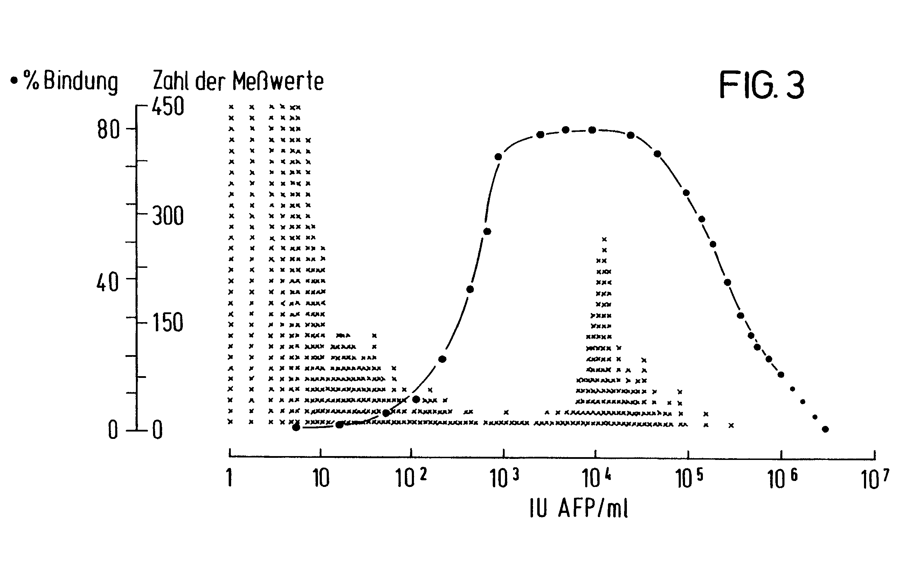

In Fig. 3 ist eine repräsentative Frequenzverteilung von Routine-AFP-Meßdaten dargestellt. Es wird deutlich, daß mit weitem Abstand die meisten Meßwerte im Bereich bis 1000 IU AFP/ml gefunden werden und nur einige wenige größer 100.000 IU AFP/ml. Zusätzlich ist in diese Verteilung die Dosis-Wirkungs-(Konzentration/Signal)-Beziehung für ein entsprechend optimiertes AFP-Assay-System eingezeichnet.

Wegen dieses Zusammenhanges wird der Standardmeßbereich in den Kernmeßbereich (0 - 150 IU AFP/ml) und den erweiterten Meßbereich (150 - 600 IU AFP/ml) unterteilt. In den Kernmeßbereich fallen mehr als 90 % aller unverdünnten Tumorproben. Der erweiterte Meßbereich ist gültig für Tumorproben, die 1 : 100 verdünnt sind (ca. 98 % dieser Proben liegen unter 600 IU AFP/ml), und für Schwangerschaftsproben (Serum oder 1 : 100 verdünnte Fruchtwasserproben; hier liegen über 99 % unter 600 IU AFP/ml).

Aufgrund der beschriebenen Erwartungswerte kann der für die unterschiedlichen Probenarten (Tumor verdünnt/unverdünnt; Schwangerschaft Serum/verdünnte Fruchtwasserproben) uneingeschränkt nutzbare Meßbereich in der angegebenen Weise definiert werden.

Arbeitsvorschrift für die erste Messung:

(Es können die Komponenten des kommerziellen Testbestecks RIA-gnost AFP c.t. (Behringwerke, Marburg) eingesetzt werden.) In eine genügende Anzahl von beschichteten Test-Röhrchen wird 20 $\mu$l Standard (bzw. Patientenprobe) pipettiert. Dabei ist für jede Probe eine neue Pipettenspitze einzusetzen. 200 $\mu$l Jod-125-anti-AFP werden in jedes Röhrchen dispersiert. Diese beiden Schritte können durch ein Dispensor/Dilutor-System automatisiert werden. Die Röhrchen werden anschließend 2 Stunden bei 17 - 27°C geschüttelt. Danach wird in jedes Röhrchen 1 ml Waschpuffer gegeben, dekantiert (abgesaugt) und mit 1 ml nachgewaschen. Die Röhrchen werden dann 1 Minute im Gammazähler gemessen und über ein gängiges Auswerte-Verfahren (z.B. Spline-Interpolation) ausgewertet.

5

Kernmeßbereich: 0 - 150 IU AFP/ml
Erweiterter Meßbereich: 150 - 600 IU AFP/ml
Referenzprobe: 100 IU AFP/ml

Tabelle 1:

Standardkurve

Gesamtaktivität:   125 774

| Standard | S0 | (= IU AFP/ml) |        | 135 cpm |
|----------|-----|--------|--------|---------|
|          | S1  | (  5   | " ) | 793 cpm |
|          | S2  | ( 15   | " ) | 2 089 cpm |
|          | S3  | ( 50   | " ) | 6 715 cpm |
|          | S4  | (100   | " ) | 12 711 cpm |
|          | S5  | (200   | " ) | 24 804 cpm |
|          | S6  | (600   | " ) | 69 997 cpm |

Patientenproben:

| 648 | 12 642 cpm = | 99.4 IU AFP/ml |
|-----|---------------|----------------|
| 649 | 7 750 cpm = | 58.6 " |
| 650 | 114 006 cpm = | >600 " |
| 651 | 101 846 cpm = | >600 " |
| 652 | 19 698 cpm = | 157.8 " |
| 653 | 51 900 cpm = | 439.8 " |
| 654 | 118 155 cpm = | >600 " |
| 655 | 3 320 cpm = | 24.3 " |
| 656 | 12 535 cpm = | 98.5 " |
| 657 | 118 787 cpm = | >600 " |
| 658 | 113 542 cpm = | >600 " |
| 659 | 3 328 cpm = | 24.3 " |
| 660 | 27 427 cpm = | 223 " |
| 661 | 282 cpm = | 1.1 " |
| 662 | 36 432 cpm = | 302 " |

Kernmeßbereich: Proben Nr. 648, 649, 655, 656, 659, 661. Diese Ergebnisse bedürfen keiner weiteren Überprüfung. Erweiterter Meßbereich: Proben Nr. 652, 653, 660, 662. Diese Proben müssen im "high-dose-hook-(HDH)"-Test eingesetzt werden. Außerhalb des Meßbereichs: Proben Nr. 650, 651, 654, 657, 658. Diese Proben müssen in den Meßbereich verdünnt werden.

1.a) Arbeitsvorschrift für den "high-dose-hook-(HDH)"-Test (2. Messung)

Es wird analog der ersten Messung gearbeitet mit der Abweichung, daß anstelle der Standardreihe nur die Referenzprobe (z.B. Testserum die ersten Messung) eingesetzt wird und anstelle von 20 $\mu$l Probenvolumen 50 $\mu$l in die Röhrchen gegeben wird. Die Inkubationszeit wird auf 10 Minuten reduziert. Die Auswertung erfolgt durch Vergleich mit der Referenzprobe 100 IU AFP/ml: Liegt die Patientenprobe im Signal (cpm) unterhalb der Referenz, so ist das in der ersten Messung erhaltene Ergebnis durch den HDH-Effekt verfälscht, liegt der cpm-Wert oberhalb, so ist das in der ersten Messung erhaltene Ergebnis bestätigt.

```
Referenz R (100 IU AFP/ml)        31 556 cpm


Patientenproben:
652                        1 634 cpm +++ HDH
653                        3 497 cpm +++ HDH
660                       34 848 cpm ---
662                       39 997 cpm ---
```

Bei den Proben 652 und 653 liegt ein HDH-Effekt vor. Durch Verdünnung in dem Meßbereich wurden die Gehalte wie folgt ermittelt: Probe 652: 600 000 IU AFP/ml; Probe 653: 200 000 IU AFP/ml.

Die Proben Nr. 660 und 662 sind HDH-negativ und somit ist das ursprünglich erhaltene Ergebnis der ersten Messung bestätigt: Probe 660: 223 IU AFP/ml und Probe 662: 302 IU AFP/ml.

1.b) Nachweis der hochkonzentrierten Proben durch Zugabe von Antigen
1. Messung wie in Beispiel 1

```
Tabelle 2:


Standardreihe
          SO (O IU AFP/ml)              215 cpm
          S1 (4.5      "    )           841 cpm
          S2 (22       "    )         3 223 cpm
          S3 (88       "    )        11 767 cpm
          S4 (440      "    )        54 236 cpm
          S5 (840      "    )        90 153 cpm
```

Patientenproben:

| | | | |
|---|---|---|---|
| 79 | 1 735 cpm | 10.4 IU AFP/ml | |
| 80 | 15 280 cpm | 115 | " |
| 81 | 4 458 cpm | 32 | " |
| 82 | 19 125 cpm | 145 | " |
| 83 | 28 665 cpm | 221 | " |
| 84 | 21 633 cpm | 165 | " |
| 85 | 83 844 cpm | 755 | " |
| 86 | 87 969 cpm | 809 | " |
| 87 | 91 833 cpm | >840 | " |
| 88 | 92 335 cpm | >840 | " |
| 89 | 92 797 cpm | >840 | " |
| 90 | 93 035 cpm | >840 | " |
| 91 | 91 933 cpm | >840 | " |
| 92 | 84 541 cpm | 764 | " |
| 93 | 75 772 cpm | 657 | " |
| 94 | 71 703 cpm | 613 | " |
| 95 | 50 732 cpm | 408 | " |
| 96 | 43 110 cpm | 341 | " |
| 97 | 33 238 cpm | 258 | " |
| 98 | 27 673 cpm | 213 | " |
| 99 | 21 081 cpm | 161 | " |

Eindeutige Resultate ergeben sich für die Proben 79, 80, 81, 82 (im Kernmeßbereich) und die Proben 87 - 91 außerhalb des Meßbereiches.

Die Proben 83, 84, 85, 86, 92 - 99 liegen im erweiterten Meßbereich und sind einem nachfolgenden "high-dose-hook-(HDH)"-Test zu unterziehen.

Arbeitsvorschrift für den HDH-Test

Es wird wie bei der ersten Messung gearbeitet mit dem Unterschied, daß zu dem Inkubationsgemisch 10 $\mu$l einer Probe von 2000 IU AFP/ml zugefügt wird.

Tabelle 3:

Patientenproben:

| 83 | 91 924 cpm --- HDH | |
|----|----|----|
| 84 | 91 561 cpm --- HDH | |
| 85 | 92 121 cpm --- HDH | |
| 86 | 90 904 cpm --- HDH | |
| 92 | 84 541 cpm +++ HDH | 50 000* |
| 93 | 74 772 cpm +++ HDH | 75 000 |
| 94 | 71 289 cpm +++ HDH | 100 000 |
| 95 | 53 278 cpm +++ HDH | 200 000 |
| 96 | 35 009 cpm +++ HDH | 300 000 |
| 97 | 31 737 cpm +++ HDH | 400 000 |
| 98 | 27 436 cpm +++ HDH | 500 000 |
| 99 | 18 466 cpm +++ HDH | 650 000 |

* IU AFP/ml durch Verdünnung bestimmt.

Beispiel 2

Radioimmunologische Bestimmung von h-Choriongonadotropin (hCG) in Patientenserum mit Hilfe eines auf monoklonalen Antikörpern basierenden 2-seitigen IRMA und der Technik der 2-stufigen Verfahrensdurchführung

Ebenso wie AFP besitzt hCG Bedeutung in zwei Indikationsgebieten:

1. Schangerschaftsverlaufskontrolle zur frühzeitigen Feststellung eines drohenden Abortes

2. Tumormarker bei trophoblastischen Tumoren

Die Normalwerte für die Schwangerschaftsverlaufskontrolle überstreichen den Bereich von ca. 100 - 250 000 mIU hCG/ml, während in der Onkologie tumormarker-negative Proben kleiner 10 und positive Proben 1000 000 mIU hCG/ml erreichen können.

Daraus ergibt die Forderung, die Schwangeren-Proben auf 1/200 vorzuverdünnen und die Tumorproben im Original einzusetzen.

Es ergibt sich dann ein Kernmeßbereich von 0 - 400 mIU hCG/ml, in welchem in der Routinediagnostik die meisten Proben gefunden werden, und ein erweiterter Meßbereich von 400 - 1000 mIU hCG/ml, welcher durch den nachfolgenden HDH-Test abgesichert werden muß. Die Referenzprobe wird bei 300 mIU hCG/ml gewählt.

Arbeitzvorschrift für die erste Messung (Es können die Komponenten von RIA-gnost hCG c.t. (Behringwerke, Marburg) eingesetzt werden)

(Durchführung wie in Beispiel 1) Probenvolumen 20 µl; Tracervolumen 200 µl, Inkubationszeit 1 Stunde.

Tabelle 4:

Standardkurve

Gesamtaktivität:    67 232 cpm

Standard S0 ( 0 mIU hCG/ml)          99 cpm

         S1 ( 11         "    )        175 cpm

         S2 ( 33         "    )        404 cpm

         S3 ( 105        "    )      1 186 cpm

         S4 ( 300        "    )      3 335 cpm

         S5 ( 600        "    )      6 767 cpm

         S6 (1000        "    )     11 305 cpm

Patientenproben:

58                    1 511 cpm     135    (mIU hCG/ml)

59                      139 cpm      7.3        "

60                    5 700 cpm     510         "

61                      192 cpm     12.7        "

62                   41 021 cpm >1000          "

63                      669 cpm     58.2        "

64                   14 076 cpm >1000          "

65                    6 972 cpm     617         "

66                    5 110 cpm     461         "

67                    5 205 cpm     469         "

68                    4 467 cpm     404         "

Kernmeßbereich: Proben 58, 59, 61, 63
Erweiterter Meßbereich: Proben 60, 65, 66, 67, 68
Außerhalb des Meßbereichs: Proben 62, 64

Arbeitsvorschrift für den "HDH-Test":

(Durchführung wie in Beispiel 1) Probenvolumen 100 $\mu$l, Tracervolumen 200 $\mu$l, Inkubationszeit 10 min.

Referenz R (300 mIU hCG/ml)        5 380 cpm

Patientenproben:

| | | | |
|---|---|---|---|
| 60 | 9 754 | cpm --- | HDH |
| 65 | 851 | cpm +++ | HDH |
| 66 | 704 | cpm +++ | HDH |
| 67 | 605 | cpm +++ | HDH |
| 68 | 506 | cpm +++ | HDH |

Durch Verdünnung wurden die Gehalt von den HDH-positiven Proben wie folgt bestimmt:

| | | |
|---|---|---|
| 65 | 100 000 | mIU hCG/ml |
| 66 | 150 000 | " |
| 67 | 200 000 | " |
| 68 | 250 000 | " |

Beispiel 3

Radioimmunologische Bestimmung von humanem thyreoideastimulierendem Hormon (hTSH) mit Hilfe eines auf zwei monoklonalen Antikörpern basierenden 2-seitigen IRMA und der Technik der 2-stufigen Verfahrensdurchführung

Prinzip der Antigen-Äquivalenterhöhung durch anti-idiotypischen Antikörper

Arbeitsvorschrift für die erste Messung: (Es können die Komponenten von RIA-gnost TSH c.t. (Behringwerke, Marburg) eingesetzt werden)

(Durchführung wie in Beispiel 1) Probenvolumen 200 $\mu$l; Tracervolumen 100 $\mu$l; Inkubationszeit 2 Stunden.

Tabelle 5:

Gesamtaktivität:

Standard SO ( 0 µIU TSH/ml)　　　　56 cpm
　" 　 S1 ( 0.13　" 　)　　　　209 cpm
　" 　 S2 ( 0.45　" 　)　　　　553 cpm
　" 　 S3 ( 1.45　" 　)　　 1 550 cpm
　" 　 S4 ( 5　　" 　)　　 4 781 cpm
　" 　 S5 ( 5　　" 　)　　 10 976 cpm
　" 　 S6 (50　　" 　)　　 18 629 cpm

Patientenproben:

| 186 | 1 885 cpm | 1.9 | µIU TSH/ml |
| 187 | 2 518 cpm | 2.7 | " |
| 188 | 5 072 cpm | 6.0 | " |
| 189 | 9 363 cpm | 13.0 | " |
| 190 | 7 230 cpm | 9.5 | " |

Aufgestockte Proben (TSH ist zugesetzt worden)

| 1 | 12 236 | 22.4 |
| 2 | 18 436 | 50.0 |
| 3 | 20 325 | >50 |
| 4 | 20 900 | >50 |
| 5 | 21 111 | >50 |
| 6 | 20 968 | >50 |
| 7 | 15 394 | 36.7 |

Kernmeßbereich: 0 - 15 µIU TSH/ml
Erweiterter Meßbereich: 15 - 50 µIU TSH/ml
Kernmeßbereich: Proben 186 - 190
Erweiterter Meßbereich: Proben 1, 2, 7
Außerhalb des Meßbereichs: Proben 3, 4, 5, 6

Da der anti-idiotypische Antikörper im Gegensatz zum Antigen nur eine Bindungsstelle für den Antikörper aufweist, kann er sich entweder mit dem markierten oder mit dem unmarkierten Antikörper verbinden, kann aber keinen ternären Komplex ausbilden. Deshalb führt der Zusatz von anti-idiotypischem Antikörper bei niedrigen Antigenkonzentrationen zu einer Abschwächung des Meßsignals, weil für das Antigen nicht mehr ausreichende Mengen der Antikörper zur Bildung des ternären, signalgebenden Komplexes zur Verfügung stehen. Dagegen ist bei hohen Antigenkonzentrationen (Bereich C von Fig. 1) die Bildung des ternäre Komplexes ohnehin so stark behindert, daß sich der Zusatz des anti-idiotypischen Antikörpers praktisch nicht mehr auswirkt.

Arbeitsvorschrift für den HDH-Test:

Zusätzlich zu den Reaktionspartnern (200 µl Probe, 100 µl markierter Antikörper) wird 20 µl einer Lösung, die 1 mg/ml anti-idiotypischen Antikörper enthält, hergestellt gemäß U.S. Patent 4.536.477, ml hinzugefügt. Ansonsten sind alle Arbeitsschritte identisch mit der ersten Messung.

```
Proben
  1            9 633 --- HDH
  2           16 125 --- HDH
  7           15 397 +++ HDH
```

Die Probe 7, die HDH-positiv ist, wurde durch Verdünnung mit 2000 µIU TSH/ml ermittelt.

## Ansprüche

1. Immunometrisches Bestimmungsverfahren für eine mindestens zwei Antikörperbindungsstellen aufweisende antigene Substanz in einer Probe, für die eine Antigenkonzentration in einem Bereich festgestellt wurde, der bis zu den Konzentrationen reicht, die noch mit genauer Präzision gemessen werden können (erweiterter Meßbereich), bei dem man eine die antigene Substanz (a) enthaltene Flüssigkeitsprobe mit einem auf einer Festphase immobilisierten, unmarkierten, für (a) spezifischen Antikörper (b) und einem weiteren, für (a) spezifischen, markierten Antikörper (c) sequentiell oder in einem Schritt inkubiert, wobei sich ein festphasengebundener, ternärer Komplex aus (a), (b) und (c) bildet, der ein nachweisbares Signal entsprechend der Menge von (a) aussendete, wobei man in einer ersten Messung die Antigenkonzentration über den gesamten Meßbereich, bestehend aus dem Bereich, in dem bei physiologischen Proben die dose-response Zuordnung eindeutig ist (Kernmeßbereich) und dem Bereich, der bis zu den Konzentrationen reicht, die noch mit genauer Präzision gemessen werden können (erweitertem Meßbereich) ermittelt, dadurch gekennzeichnet, daß man die Probe, für die eine Antigenkonzentration im erweiterten Meßbereich festgestellt wurde, nach Erhöhung der Menge des Antigens und/oder eines Antigenäquivalents einer zweiten Messung unterwirft.

2. Verfahren nach Anspruch 1, dadurch gekennzeichnet, daß man bei der zweiten Messung zur Erhöhung der Antigenmenge ein größeres Probenvolumen einsetzt und nach einer kürzeren Inkubationszeit als bei der ersten Messung die Antigenmenge feststellt.

3. Verfahren nach Anspruch 1, dadurch gekennzeichnet, daß man vor der zweiten Messung die Konzentration des Antigens durch Zusatz weiterer Antigenmengen und/oder eines anti-idiotypischen Antikörpers erhöht.

4. Verfahren nach Anspruch 1, dadurch gekennzeichnet, daß der auf der Festphase immobilisierte, unmarkierte Antikörper (b) ein monoklonaler oder polyklonaler Antikörper ist.

5. Verfahren nach Anspruch 4, dadurch gekennzeichnet, daß der unmarkierte Antikörper (b) an die Festphase adsorptiv oder kovalent gebunden ist.

6. Verfahren nach Anspruch 1, dadurch gekennzeichnet, daß die Festphase aus einem Kunststoffröhrchen, Mikrotiter-Platten, Kunststoffkugeln oder -propellern oder aus mikroskopisch kleinen Kunststoffkugeln besteht, die in einer Flüssigkeit suspendiert sind.

7. Verfahren nach Anspruch 1, dadurch gekennzeichnet, daß der markierte Antikörper (c) ein monoklonaler oder polyklonaler Antikörper ist und ein radioaktives Isotop, ein Enzym, eine fluoreszierende oder eine chemilumineszierende Gruppe als Indikator trägt.

8. Verfahren nach Anspruch 1, dadurch gekennzeichnet, daß als antigene Substanzen das humane thyreoideastimulierende Hormon (hTSH) oder die onkofetalen Proteine Alfa-Fetoprotein, h-Choriongonadotropin oder das carcino-embryonale Antigen bestimt werden.

## Claims

1. An immunometric determination method for an antigenic substance having at least two antibody binding points in a sample for which an antigen concentration in a range which can still be measured with accurate precision (extended measurement range) is determined, in which method a liquid sample containing the antigenic substance (a) is incubated sequentially or in one step with an unlabeled antibody (b) which is specific for (a) and which is immobilized on a solid phase, and a further labeled antibody (c) which is specific for (a), a solid phase-bound, ternary complex of (a), (b) and (c) being formed which emitted a detectable signal corresponding to the amount of (a), where, in a first measurement, the antigen concentration is determined over the entire measurement range, comprising the range in which the dose-response allocation in the physiological sample is unambiguous (core measurement range) and the range which extends up to the concentrations which can still be measured with accurate precision (extended measurement range), wherein the sample for which an antigen concentration has been determined in the extended measurement range is subjected to a second measurement after the amount of the antigen and/or an antigen equivalent has been increased.

2. The method as claimed in claim 1, wherein, in the second measurement, a larger volume of sample is employed in order to increase the amount of antigen, and the amount of antigen is determined after a shorter incubation time than in the first measurement.

3. The method as claimed in claim 1, wherein the concentration of the antigen is increased before the second measurement by adding further amounts of antigen and/or an antiidiotypic antibody.

4. The method as claimed in claim 1, wherein the unlabeled antibody (b) immobilized on the solid phase is a monoclonal or polyclonal antibody.

5. The method as claimed in claim 4, wherein the unlabeled antibody (b) is bound adsorptively or covalently to the solid phase.

6. The method as claimed in claim 1, wherein the solid phase comprises plastic tubes, microtiter plates, plastic beads or propellers or microscopically small plastic beads which are suspended in a liquid.

7. The method as claimed in claim 1, wherein the labeled antibody (c) is a monoclonal or polyclonal antibody and carries a radioactive isotope, an enzyme, a fluorescent group or a chemiluminescent group as indicator.

8. The method as claimed in claim 1, wherein the antigenic substances determined are human thyroid stimulating hormone (hTSH) or the oncofetal proteins alpha-feto-protein, human chorionic gonadotropin or carcinoembryonic antigen.

## Revendications

1. Procédé de dosage immunométrique d'une substance antigène présentant au moins deux sites de fixation d'anticorps dans un échantillon pour lequel on a déterminé une concentration d'antigène dans un domaine qui atteint les concentrations qu'on peut encore mesurer avec une précision suffisante (domaine de mesure élargi), au cours duquel on fait incuber, successivement ou en une seule étape, un échantillon liquide contenant la substance antigène (a) avec un anticorps (b) spécifique de (a), immobilisé sur une phase solide, non marqué, et un autre anticorps (c) spécifique de (a), marqué grâce à quoi un complexe ternaire, lié à la phase solide, se forme à partir de (a), (b) et (c), qui émet un signal détectable correspondant à la quantité de (a), moyennant quoi on détermine, par une première mesure, la concentration d'antigène dans l'ensemble du domaine de mesure, composé du domaine dans lequel la relation dose-réponse est univoque pour des échantillons physiologiques (domaine de mesure restreint) et du domaine qui atteint les concentrations qu'on peut encore mesurer avec une précision

14

suffisante (domaine de mesure élargi), caractérisé en ce que l'on soumet l'échantillon, pour lequel on a determiné une concentration d'antigène dans le domaine de mesure élargi, à une deuxième mesure après augmentation de la quantité d'antigène et/ou d'un équivalent antigène.

2. Procédé selon la revendication 1, caractérisé en ce qu on met en oeuvre un volume d'échantillon plus grand au cours de la deuxième mesure, pour augmenter la quantité d'antigène, et on détermine la quantité d'antigène après une durée d'incubation plus courte que pour la première mesure.

3. Procédé selon la revendication 1, caractérisé en ce qu'on augmente la concentration d'antigène, avant la deuxième mesure, par addition de quantités supplémentaires d'antigène et/ou d'un anticorps antiidiotypique.

4. Procédé selon la revendication 1, caractérisé en ce que l'anticorps (b) non marqué, immobilisé sur la phase solide, est un anticorps monoclonal ou polyclonal.

5. Procédé selon la revendication 4, caractérisé en ce que l'anticorps (b) non marqué est lié à la phase solide par adsorption ou par covalence.

6. Procédé selon la revendication 1 caractérisé en ce que la phase solide est constituée d'un tube en matière plastique, de plaquettes de microtitrage, de sphères ou d'hélices en matière synthétique, ou encore de petites billes microscopiques en matière synthétique qui sont en suspension dans un liquide.

7. Procédé selon la revendication 1, caractérisé en ce que l'anticorps (c) marqué est un anticorps monoclonal ou polyclonal et porte un isotope radioactif, une enzyme, un groupement fluorescent ou chimiluminescent comme indicateur.

8. Procédé selon la revendication 1, caractérisé en ce que l'on dose comme substances antigènes l'hormone humaine de stimulation de la thyroïde (hTSH) ou les protéines onco-foetales alpha-foeto-protéine, l'hormone chorionique gonadotrope humaine ou l'antigène carcino-embryonnaire (CEA).

## FIG. 1

FIG.2

$V_1 = 10\,\mu l$
$V_2 = 20\,\mu l$
$V_5 = 50\,\mu l$

EP 0 263 401 B1

FIG.3

Zahl der Meßwerte

● % Bindung

IU AFP/ml